# EUROPEAN PATENT APPLICATION

(11) **EP 4 338 658 A1**
(43) Date of publication of application: **20.03.2024**
(21) Application number: 22842090.7
(22) Date of filing: 11.07.2022
(51) Int. Cl.: A61B 3/117

(54) **OCULAR OPTICAL LENS, LIGHT MEASURING SYSTEM, AND LIGHT MEASURING METHOD**

(30) Priority: 14.07.2021 JP 2021116647
(71) Applicant: Seed Co., Ltd., Tokyo 113-8402 (JP); Keio University, Tokyo, 108-8345 (JP); Centuryarks Co., Ltd., Tokyo 106-0031 (JP)
(72) Inventor: SAWADA, Mio, Tokyo 113-8402 (JP); KINOSHITA, Taku, Tokyo 113-8402 (JP); KUBOTA, Shin, Kounosu-shi, Saitama 369-0131 (JP); KURIHARA, Toshihide, Tokyo 160-8582 (JP); SHINOJIMA, Ari, Tokyo 160-8582 (JP); FURUYA, Manabu, Tokyo 106-0031 (JP)
(74) Representative: Prüfer & Partner mbB Patentanwälte · Rechtsanwälte
(86) International application number: PCT/JP2022/027306
(87) International publication number: WO 2023/286746

(57) **Abstract**

An eyepiece optical lens includes: a base that has a plurality of faces; a concave portion that forms one face of the base, and is concave in a spherical shape; and a reflection portion that forms a face on an outer peripheral side of the concave portion of the base, the reflection portion reflecting, toward the concave portion, light that has been made incident on the base from a face that is included in the plurality of faces and is different from the concave portion. This provides an eyepiece optical lens that enables a non-invasive analysis of substances contained in aqueous humor of a subject eye.

## Description

### Field

The present invention relates to an eyepiece optical lens, a light measurement system, and a light measurement method.

### Background

There has been conventionally known a technique of analyzing substances contained in aqueous humor in a subject eye to assist in diagnosis of the subject eye. In this technique, it is necessary to perform paracentesis to extract aqueous humor below the cornea, and this has imposed a large burden on subjects.

Patent Literatures 1 to 4 disclose a technique for conducting a non-invasive examination on a subject eye. In these techniques, light is made transversely incident from a nose side or an ear side of the subject eye, and the light is transmitted in aqueous humor along a direction that is roughly orthogonal to a direction of an eye axis (a line connecting an apex of the cornea to the fovea centralis) of the subject eye. In this case, the light is transmitted through the anterior cornea, the aqueous humor, and the posterior cornea of the subject eye in this order. It has been proposed that this light path causes a reduction in the number of tissues to be transmitted, which are noise sources, and the concentration of a metabolite of the aqueous humor is measured. Furthermore, this light path prevents light from passing through the pupil, entering an inside of the eyeball, and damaging the retina.

### Citation List

### Patent Literature

Patent Literature 1: JP H6-503245 A
Patent Literature 2: JP 2018-175760 A
Patent Literature 3: JP 2018-175481 A
Patent Literature 4: JP 2011-83342 A

### Summary

### Technical Problem

However, according to results of anatomical researches conducted by the present inventors on a shape of a corneal ring and a trial calculation based on data relating to the divergence of a light flux to be introduced or a movement of the eyeball, in order to achieve a light path that causes transverse transmission through aqueous humor in eyes of many subjects, it is necessary to control an angle of incidence of light on a subject eye with an error that is smaller than 1°, and it is considered difficult to achieve the light path.

The present invention has been made in view of the above, and provides an eyepiece optical lens, a light measurement system, and a light measurement method that enable a non-invasive analysis of substances contained in aqueous humor of a subject eye.

### Solution to Problem

To solve the problem described above and to achieve the object, an eyepiece optical lens according to the present invention includes: a base that has a plurality of faces; a concave portion configured to form one face of the base, the concave portion being concave in a spherical shape; and a reflection portion configured to form a face on an outer peripheral side of the concave portion of the base, the reflection portion reflecting, toward the concave portion, light that has been made incident on the base from a face that is included in the plurality of faces and is different from the concave portion.

In the eyepiece optical lens according to one aspect of the present invention, the reflection portion is configured to reflect the light that has been made incident on the base, in a direction that is roughly orthogonal to an incident direction.

In the eyepiece optical lens according to one aspect of the present invention, the reflection portion is configured to reflect the light that has been made incident on the base, in an axial direction that passes through an apex of the concave portion.

In the eyepiece optical lens according to one aspect of the present invention, the reflection portion has a critical angle that is determined according to a refractive index of a medium forming the eyepiece optical lens, the critical angle causing light made incident on the reflection portion to be totally reflected.

In the eyepiece optical lens according to one aspect of the present invention, the reflection portion is configured to form a face surrounding an outer periphery of the concave portion of the base.

In the eyepiece optical lens according to one aspect of the present invention, the reflection portion includes: a first reflection portion configured to form the face on the outer peripheral side of the concave portion of the base, the first reflection portion reflecting, toward the concave portion, the light that has been made incident on the base from the face that is included in the plurality of faces and is different from the concave portion; and a second reflection portion configured to form the face of the outer peripheral side of the concave portion of the base, the second reflection portion reflecting the light that has been reflected by the first reflection portion, toward the face that is included in the plurality of faces and is different from the concave portion.

A light measurement system according to one aspect of the present invention includes: the above-mentioned eyepiece optical lens; and a light measurement apparatus that includes a light source unit configured to irradiate the eyepiece optical lens with light, and a light receiver configured to receive the light from the eyepiece optical lens.

In the light measurement system according to one aspect of the present invention, the light receiver is configured to receive Raman scattered light in aqueous humor of a subject eye that has been brought into contact with the eyepiece optical lens.

A light measurement method according to one aspect of the present invention is a light measurement method that uses an eyepiece optical lens including: a base that has a plurality of faces; a concave portion configured to form one face of the base, the concave portion being concave in a spherical shape; and a reflection portion configured to form a face on an outer peripheral side of the concave portion of the base, the reflection portion reflecting, toward the concave portion, light that has been made incident on the base from a face that is included in the plurality of faces and is different from the concave portion, the light measurement method including: an adjustment step of irradiating a subject eye with examination light, and adjusting an incident position of the light to cause a position of a spot of the examination light to be formed in the reflection portion, the subject eye having been brought into contact with the eyepiece optical lens; and a measurement light irradiation step of irradiating the eyepiece optical lens with measurement light having a light intensity that is greater than a light intensity of the examination light; and a light reception step of receiving the measurement light.

The light measurement method according to one aspect of the present invention further includes, before the light reception step, a disposition step of disposing a light receiver configured to receive the measurement light, in a position that causes Raman scattered light in aqueous humor of the subject eye to have a maximum light intensity. Advantageous Effects of Invention

The present invention can achieve an eyepiece optical lens, a light measurement system, and a light measurement method that enable a non-invasive analysis of substances contained in aqueous humor of a subject eye.

### Brief Description of Drawings

FIG. 1 is a schematic diagram of a Raman scattered light spectroscopic measurement system according to an embodiment of the present invention.
FIG. 2 is an enlarged view of an eyepiece optical lens.
FIG. 3 is a partial enlarged view of a light source unit.
FIG. 4 is a flowchart illustrating a procedure of a Raman spectroscopic measurement method.
FIG. 5 is an enlarged view of an eyepiece optical lens of a first variation.
FIG. 6 is an enlarged view of a light source unit of a second variation.
FIG. 7 is an enlarged view of a light source unit of a third variation.

### Description of Embodiments

Hereinafter, details of an eyepiece optical lens, a light measurement system, and a light measurement method of the present invention will be described on the basis of embodiments, but these are not restrictive of the present invention.

### (Embodiment)

### [Configuration of Raman Scattered Light Spectroscopic Measurement System]

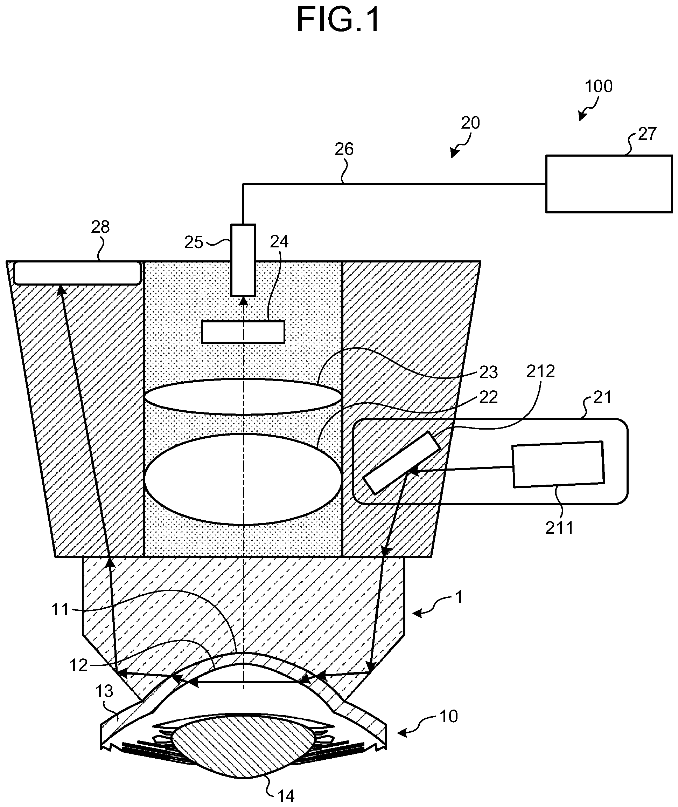

FIG. 1 is a schematic diagram of a Raman scattered light spectroscopic measurement system according to an embodiment of the present invention. A subject eye 10 includes a cornea 11 that takes light into the eyeball, aqueous humor 12 that is located inside the cornea 11, and adjusts intraocular pressure, a sclera 13 that is located on an outer periphery of the cornea 11, and a crystalline lens 14 that is located inside the aqueous humor 12. The aqueous humor 12 contains a variety of metabolites, and it is considered that there is a correlation between these substances and eye diseases. Therefore, there is a request for a technique for identifying metabolites in the aqueous humor 12 and measuring the concentrations of the metabolites. A Raman scattered light spectroscopic measurement system 100 performs measurement for analyzing the metabolites contained in the aqueous humor 12 by using Raman spectroscopy. The Raman scattered light spectroscopic measurement system 100 includes an eyepiece optical lens 1 that is brought into contact with the subject eye 10, and a Raman scattered light spectroscopic measurement apparatus 20 that irradiates the eyepiece optical lens 1 with pumping light, and performs spectroscopic measurement on Raman scattered light from the aqueous humor 12 of the subject eye 10, as illustrated in FIG. 1. Note that in the present invention, "contact" refers to a state where the eyepiece optical lens 1 of the present invention comes into contact with the cornea 11, and indicates a state that is similar to "wear", "mount", "attach", "abut", or the like.

### [Configuration of Eyepiece Optical Lens]

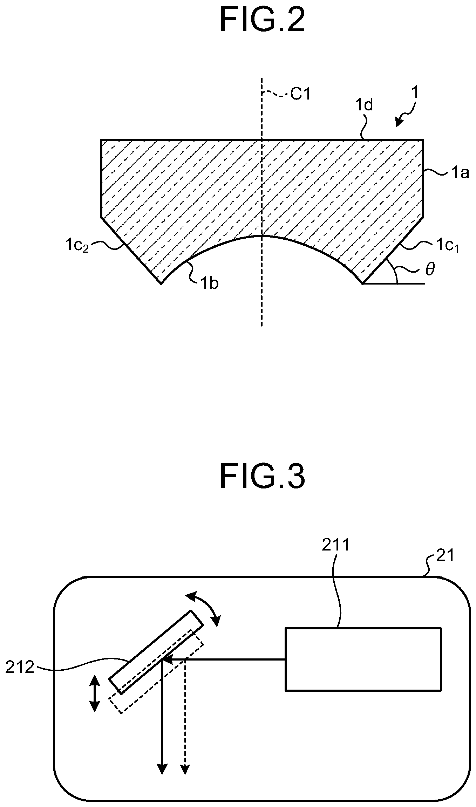

FIG. 2 is an enlarged view of an eyepiece optical lens. FIG. 2 illustrates a cross section of the eyepiece optical lens 1, and the eyepiece optical lens 1 includes a base 1a that has a plurality of faces, a concave portion 1b that forms one face of the base 1a, a reflection portion 1c that forms a face on an outer peripheral side of the concave portion 1b of the base 1a, and an incidence portion 1d that forms a face on an opposite side of the concave portion 1b. Note that herein, a face on a side of the incidence portion 1d (on an upper side in FIG. 2) of the base 1a is referred to as a front face, and a face on a side of the concave portion 1b (on a lower side in FIG. 2) of the base 1a is referred to as a rear face.

It is preferable that a refractive index of the eyepiece optical lens 1 be greater than a refractive index (1.00) of the air, and be also greater than a refractive index (1.376) of the cornea 11. Furthermore, it is preferable that the eyepiece optical lens 1 have such hardness that a radius of curvature of the base 1a does not change in a case where the eyepiece optical lens 1 abuts onto the cornea 11. As a material that satisfies these conditions, the eyepiece optical lens 1 is made of, for example, glass, poly methyl methacrylate (PMMA), or the like.

The concave portion 1b is concave in a spherical shape, and has a radius of curvature that corresponds to the cornea 11 of the subject eye 10. It is preferable that the radius of curvature of the concave portion 1b be equal to a radius of curvature of the cornea 11, or be greater than the radius of curvature of the cornea 11. The concave portion 1b is formed to have such a radius of curvature, and this can prevent a shape of the subject eye 10 from changing during measurement after the eyepiece optical lens 1 has been brought into contact with the subject eye 10.

The reflection portion 1c (a face on which a reflection portion 1c₁ and a reflection portion 1c₂ are continuously formed) is, for example, an interface between glass and the air, and has a critical angle that is determined according to a refractive index of a medium that forms the eyepiece optical lens 1, and causes light made incident on the reflection portion 1c to be totally reflected. However, it is sufficient if the reflection portion 1c is a reflection portion having a sufficiently high reflectance, and the reflection portion 1c is not limited to a total reflection type. Furthermore, the reflection portion 1c may be formed by forming a thin film that is made of metal or the like on a surface of glass. The reflection portion 1c is formed in an annular shape to surround an outer periphery of the concave portion 1b. In other words, a cross section that passes through an apex of the concave portion 1b, and includes a center axis C1 that passes through a center of a spherical surface formed by the concave portion 1b, as illustrated in FIG. 2, has the same shape in any direction. Note that the apex of the concave portion 1b means a point where the concave portion 1b is most concave.

Returning to FIG. 2, the reflection portion 1c₁ reflects light that has been made incident on the base 1a from the incidence portion 1d, toward the center axis C1 in a direction that is roughly orthogonal to an incident direction. However, it is sufficient if the reflection portion 1c₁ reflects, toward the concave portion 1b, the light that has been made incident on the base 1a from the incidence portion 1d, and it is sufficient if light reflected by the reflection portion 1c₁ passes through a portion of the aqueous humor 12. An angle θ formed by the reflection portion 1c₁ relative to a leftward/rightward direction of the subject eye 10 (a leftward/rightward direction in FIG. 2) is an angle at which light that has been made incident from the front is reflected at roughly right angles to the incident direction, and therefore the angle θ is, for example, 45°. However, the angle θ can be appropriately selected according to a direction in which light is made incident on the subject eye 10. For example, in a case where light is made incident on the subject eye 10 to be inclined outward (to a right-hand side in FIG. 2) from the center axis C1 of the concave portion 1b, the angle θ formed by the reflection portion 1c₁ may be set in such a way that reflected light goes straight to the center axis C1.

Furthermore, the reflection portion 1c₂ reflects, toward the incidence portion 1d, light that has been reflected by the reflection portion 1c₁ and has passed through the aqueous humor 12. An angle formed by the reflection portion 1c₂ relative to the leftward/rightward direction of the subject eye 10 is an angle at which light from the concave portion 1b is reflected forward, and therefore the angle is, for example, 45°. However, the angle can be appropriately selected according to a direction in which light is measured.

It is preferable that the incidence portion 1d have a small reflectance with respect to light from the front, and for example, a flat plane that is orthogonal to the center axis C1 is formed.

### [Configuration of Raman Scattered Light Spectroscopic Measurement Apparatus]

The Raman scattered light spectroscopic measurement apparatus 20 includes a light source unit 21 that irradiates the eyepiece optical lens 1 with light, an objective 22, a condenser lens 23, a notch filter 24, an optical fiber coupler 25, an optical fiber 26, a spectroscope 27 serving as a light receiver that receives light from the eyepiece optical lens 1, and an absorber 28, as illustrated in FIG. 1.

The Raman scattered light spectroscopic measurement apparatus 20 measures Raman scattered light that has been scattered in a direction that is roughly orthogonal to light that the concave portion 1b has introduced into the aqueous humor 12, by using, as pumping light, incident light that has been applied from the light source unit 21. This is because Raman scattered light has the same light intensity in any direction of measurement, while Rayleigh scattered light has the smallest light intensity in a direction that is roughly orthogonal to the pumping light, and this enables Raman scattered light to be efficiently measured.

FIG. 3 is a partial enlarged view of a light source unit. As illustrated in FIG. 3, the light source unit 21 includes a light source 211 that irradiates the subject eye 10 with light, and a MEMS mirror 212 that can change in a reflection direction.

The light source 211 emits measurement light that is used as pumping light for performing spectroscopic measurement on Raman scattered light in the aqueous humor 12 of the subject eye 10. Furthermore, the light source 211 emits examination light having a light intensity that is smaller than a light intensity of this measurement light. The examination light is used to examine whether the reflection portion 1c has been irradiated with light from the light source 211 before measurement. Wavelengths of the measurement light and the examination light are, for example, 532 nm, but are not particularly limited. It is preferable that the examination light have the same wavelength as a wavelength of the measurement light. It is sufficient if the light source 211 is a light source that emits light of a predetermined wavelength, and an example is a laser diode (LD).

The objective 22 condenses scattered light from the aqueous humor 12.

The condenser lens 23 couples light that has been condensed by the objective 22 to the optical fiber coupler 25.

The notch filter 24 is a band-stop filter that selectively shields Rayleigh scattered light included in the scattered light from the aqueous humor 12. Accordingly, the notch filter 24 shields Rayleigh scattered light included in the scattered light from the aqueous humor 12, and transmits Raman scattered light. However, it is sufficient if the notch filter 24 is a filter that selectively shields light of the same wavelength as a wavelength of the Rayleigh scattered light included in the scattered light from the aqueous humor 12, and transmits Raman scattered light included in the scattered light from the aqueous humor 12. For example, in a case where Stokes Raman scattering is measured, a filter that transmits light of a wavelength that is longer than a wavelength of pumping light applied by the light source unit 21 may be used. Furthermore, in a case where anti-Stokes Raman scattering is measured, a filter that transmits light of a wavelength that is shorter than the wavelength of the pumping light applied by the light source unit 21 may be used. Moreover, in a case where Raman scattered light of a known wavelength is measured, a bandpass filter that selectively transmits Raman scattered light may be used.

The optical fiber coupler 25 couples light that has been condensed by the condenser lens 23 to the optical fiber 26.

The optical fiber 26 introduces scattered light from the aqueous humor 12 into the spectroscope 27.

The spectroscope 27 performs spectroscopic measurement on light that has been transmitted through the notch filter 24. Note that light that has been condensed by the condenser lens 23 may be made directly incident on the spectroscope. In this case, the optical fiber coupler 25 and the optical fiber 26 are omitted.

The absorber 28 absorbs light that has been applied by the light source unit 21.

[Raman Scattered Light Spectroscopic Measurement Method Performed by Raman Scattered Light Spectroscopic Measurement System]

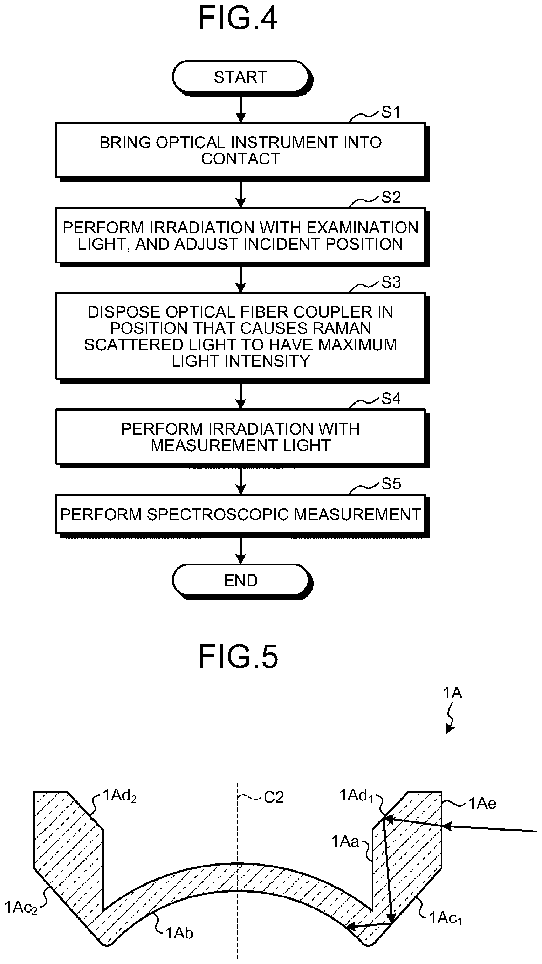

Next, a Raman scattered light spectroscopic measurement method, which is a light measurement method performed by the Raman scattered light spectroscopic measurement system 100, is described. FIG. 4 is a flowchart illustrating a procedure of the Raman spectroscopic measurement method. As illustrated in FIG. 4, the eyepiece optical lens 1 is brought into contact with the subject eye 10 (Step S1).

Next, the subject eye 10 that has come into contact with the eyepiece optical lens 1 is irradiated with examination light from the light source unit 21. Then, an incident position of light is adjusted in such a way that a spot position of the examination light is formed in the reflection portion 1c₁ (Step S2: adjustment step). Note that if an incident direction and the spot position of light are confirmed and a refractive index of each substance that light passes through is used, a light path can be estimated according to the Snell's law. Stated another way, an incident position of light is adjusted while a position of a spot is confirmed, and this enables a light path to be adjusted in such a way that incident light transversely passes through the aqueous humor 12 of the subject eye 10.

Moreover, the optical fiber coupler 25 that introduces light into the spectroscope 27 serving as a light receiver is disposed in a position that causes Raman scattered light in the aqueous humor 12 of the subject eye 10 to have a maximum light intensity (Step S3: disposition step).

Then, the eyepiece optical lens 1 is irradiated from the light source unit 21 with measurement light having a light intensity that is greater than a light intensity of the examination light (Step S4: measurement light irradiation step).

Then, the measurement light is introduced from the optical fiber coupler 25 into the spectroscope 27, and spectroscopic measurement is performed by the spectroscope 27 (Step S5: light reception step).

According to the Raman scattered light spectroscopic measurement method described above, by using the eyepiece optical lens 1 including the concave portion 1b, light can be made incident on the subject eye 10 from the front, and substances contained in the aqueous humor 12 can be analyzed.

### [Variations of Eyepiece Optical Lens]

### (First Variation)

FIG. 5 is an enlarged view of an eyepiece optical lens of a first variation. FIG. 5 illustrates a cross section of an eyepiece optical lens 1A of the first variation, and the eyepiece optical lens 1A includes a base 1Aa that has a plurality of faces, a concave portion 1Ab that forms one face of the base 1Aa, a reflection portion 1Ac (a face on which a reflection portion 1Ac₁ and a reflection portion 1Ac₂ are continuously formed) that forms a face on an outer peripheral side of the concave portion 1Ab of the base 1Aa, a third reflection portion 1Ad (a face on which a third reflection portion 1Ad₁ and a third reflection portion 1Ad₂ are continuously formed) that forms a face that faces the reflection portion 1Ac of the base 1Aa, and an incidence portion 1Ae that forms a side face of the base 1Aa. The reflection portion 1Ac and the third reflection portion 1Ad are formed in an annular shape to surround an outer periphery of the concave portion 1Ab. In other words, a cross section that passes through an apex of the concave portion 1Ab, and includes a center axis C2 that passes through a center of a spherical surface formed by the concave portion 1Ab, as illustrated in FIG. 5, has the same shape in any direction.

The reflection portion 1Ac₁ reflects light that has been made incident from the incidence portion 1Ae and has been reflected by the third reflection portion 1Ad₁, toward the center axis C2 in a direction that is roughly orthogonal to an incident direction. Furthermore, the reflection portion 1Ac₂ reflects light that has been reflected by the reflection portion 1Ac₁, toward the third reflection portion 1Ad₂.

The third reflection portion 1Ad₁ reflects light that has been made incident on the incidence portion 1Ae from a side (a nose side or an ear side) of the subject eye 10, in a direction that is roughly parallel to the center axis C2. Furthermore, the third reflection portion 1Ad₂ reflects light that has been reflected by the reflection portion 1Ac₂, in a direction that is roughly orthogonal to the center axis C2.

As described above in the first variation, a configuration that introduces incident light from a side of the subject eye 10 may be employed. In this case, angles, positions, sizes, or the like of the reflection portion 1Ac (the face on which the reflection portion 1Ac₁ and the reflection portion 1Ac₂ are continuously formed) and the third reflection portion 1Ad (the face on which the third reflection portion 1Ad₁ and the third reflection portion 1Ad₂ are continuously formed) are appropriately set, and this enables light to be easily made incident on the aqueous humor 12 in comparison with a case where incident light is directly introduced into the aqueous humor 12 from the side.

### (Second Variation)

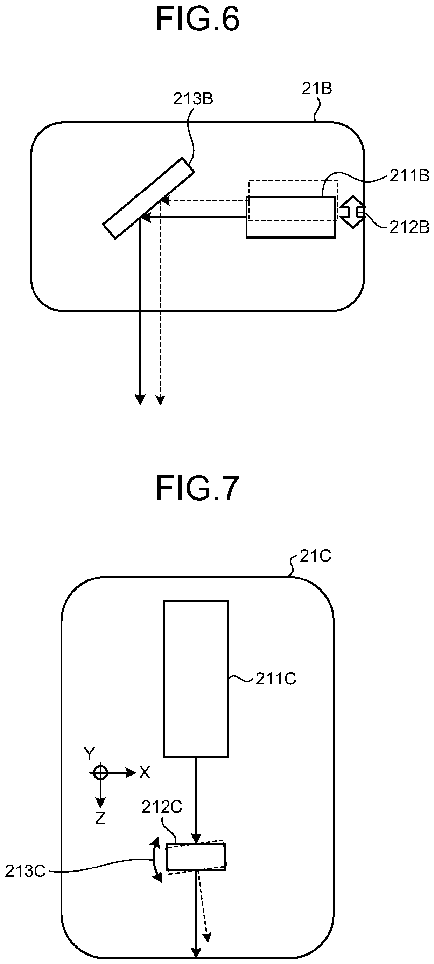

FIG. 6 is an enlarged view of a light source unit of a second variation. As illustrated in FIG. 6, a light source unit 21B includes a light source 211B that irradiates the subject eye 10 with light, a driving unit 212B that drives the light source 211B, and a mirror 213B that reflects light from the light source 211B toward the subject eye 10.

The driving unit 212B includes a rail that extends in a direction that is orthogonal to a direction in which the light source 211B applies light, and a motor that drives the light source 211B, and drives the motor to move the light source 211B that is movably held on the rail. According to a movement of the light source 211B on the rail, a position of irradiation of the eyepiece optical lens 1 with light can be adjusted.

### (Third Variation)

FIG. 7 is an enlarged view of a light source unit of a third variation. As illustrated in FIG. 7, a light source unit 21C includes a light source 211C that irradiates the subject eye 10 with light, a lens 212C, and a driving unit 213C that rotates the lens 212C to change a traveling direction of light that has been transmitted through the lens 212C.

Note that, in the embodiment, an example where the reflection portion 1c surrounds the concave portion 1b in an annular shape has been described, but this is not restrictive. The reflection portion 1c may include a first reflection portion that forms a face on an outer peripheral side of the concave portion 1b of the base 1a, and reflects light that has been made incident from the incidence portion 1d, toward the concave portion 1b, and a second reflection portion that forms a face on the outer peripheral side of the concave portion 1b of the base 1a, and reflects light that has been reflected by the first reflection portion, toward the incidence portion 1d. In other words, the reflection portion 1c may include a plurality of reflection portions that is provided to be spaced apart from each other. In this case, it is preferable that the eyepiece optical lens 1 be held to not rotate relative to the subject eye 10. Furthermore, the reflection portion 1c may only include a reflection portion that forms a face on the outer peripheral side of the concave portion 1b of the base 1a, and reflects light that has been made incident from the incidence portion 1d, toward the concave portion 1b. The formation of the reflection portion 1c, as described above, enables light made incident on the subject eye 10 from the front to be transversely introduced into the aqueous humor 12. In this case, light that has been transmitted through the aqueous humor 12 is emitted to a side (the nose side or the ear side) of the subject eye 10, and therefore the absorber 28 may be disposed on the side of the subject eye 10.

### Reference Signs List

1, 1A EYEPIECE OPTICAL LENS
1a, 1Aa BASE
1b, 1Ab CONCAVE PORTION
1c, 1Ac REFLECTION PORTION
1d, 1Ae INCIDENCE PORTION
1Ad THIRD REFLECTION PORTION
10 SUBJECT EYE
11 CORNEA
12 AQUEOUS HUMOR
13 SCLERA
14 CRYSTALLINE LENS
20 RAMAN SCATTERED LIGHT SPECTROSCOPIC MEASUREMENT APPARATUS
21, 21B, 21C LIGHT SOURCE UNIT
22 OBJECTIVE
23 CONDENSER LENS
24 NOTCH FILTER
25 OPTICAL FIBER COUPLER
26 OPTICAL FIBER
27 SPECTROSCOPE
28 ABSORBER
100 RAMAN SCATTERED LIGHT SPECTROSCOPIC MEASUREMENT SYSTEM
211, 211B, 211C LIGHT SOURCE
212 MEMS MIRROR
212B, 213C DRIVING UNIT
213B MIRROR
212C LENS

## Claims

1. An eyepiece optical lens comprising:
a base that has a plurality of faces;
a concave portion configured to form one face of the base, the concave portion being concave in a spherical shape; and
a reflection portion configured to form a face on an outer peripheral side of the concave portion of the base, the reflection portion reflecting, toward the concave portion, light that has been made incident on the base from a face that is included in the plurality of faces and is different from the concave portion.

2. The eyepiece optical lens according to claim 1,
wherein the reflection portion is configured to reflect the light that has been made incident on the base, in a direction that is roughly orthogonal to an incident direction.

3. The eyepiece optical lens according to claim 1, wherein the reflection portion is configured to reflect the light that has been made incident on the base, in an axial direction that passes through an apex of the concave portion.

4. The eyepiece optical lens according to claim 1,
wherein the reflection portion has a critical angle that is determined according to a refractive index of a medium forming the eyepiece optical lens, the critical angle causing light made incident on the reflection portion to be totally reflected.

5. The eyepiece optical lens according to claim 1,
wherein the reflection portion is configured to form a face surrounding an outer periphery of the concave portion of the base.

6. The eyepiece optical lens according to claim 1, wherein
the reflection portion includes:
a first reflection portion configured to form the face on the outer peripheral side of the concave portion of the base, the first reflection portion reflecting, toward the concave portion, the light that has been made incident on the base from the face that is included in the plurality of faces and is different from the concave portion; and
a second reflection portion configured to form the face of the outer peripheral side of the concave portion of the base, the second reflection portion reflecting the light that has been reflected by the first reflection portion, toward the face that is included in the plurality of faces and is different from the concave portion.

7. A light measurement system comprising:
the eyepiece optical lens according to claim 1; and
a light measurement apparatus that includes a light source unit configured to irradiate the eyepiece optical lens with light, and a light receiver configured to receive the light from the eyepiece optical lens.

8. The light measurement system according to claim 7, wherein the light receiver is configured to receive Raman scattered light in aqueous humor of a subject eye that has been brought into contact with the eyepiece optical lens.

9. A light measurement method that uses an eyepiece optical lens including:
a base that has a plurality of faces;
a concave portion configured to form one face of the base, the concave portion being concave in a spherical shape; and
a reflection portion configured to form a face on an outer peripheral side of the concave portion of the base, the reflection portion reflecting, toward the concave portion, light that has been made incident on the base from a face that is included in the plurality of faces and is different from the concave portion, the light measurement method comprising:
an adjustment step of irradiating a subject eye with examination light, and adjusting an incident position of the light to cause a position of a spot of the examination light to be formed in the reflection portion, the subject eye having been brought into contact with the eyepiece optical lens; and
a measurement light irradiation step of irradiating the eyepiece optical lens with measurement light having a light intensity that is greater than a light intensity of the examination light; and
a light reception step of receiving the measurement light.

10. The light measurement method according to claim 9, further comprising, before the light reception step, a disposition step of disposing a light receiver configured to receive the measurement light, in a position that causes Raman scattered light in aqueous humor of the subject eye to have a maximum light intensity.
